# EUROPEAN PATENT APPLICATION

(11) **EP 1 391 221 A1**
(43) Date of publication of application: **25.02.2004**
(21) Application number: 02018922.1
(22) Date of filing: 23.08.2002
(51) Int. Cl.: A61P 35/00, A61P 37/06, A61K 31/555, A61K 31/52, A61K 31/435, A61K 31/164, A61K 39/395, A61K 38/13

(54) **A pharmaceutical preparation containing palladium complex compounds and the uses thereof for treating cancer and autoimmune disease**

(71) Applicant: Deutsches Krebsforschungszentrum Stiftung des öffentlichen Rechts, 69120 Heidelberg (DE); Ruprecht-Karls-Universität Heidelberg, 69117 Heidelberg (DE)
(72) Inventor: Amtmann, Eberhard, D-69121 Heidelberg (DE); Friebolin, Wolfgang, D-69123 Heidelberg (DE); Schilling, Gerhard, D-68526 Ladenburg (DE)
(74) Representative: Schüssler, Andrea, Dr.

(57) **Abstract**

The present invention relates to a pharmaceutical preparation containing at least one compound of general formula (I)

Pd(S₂COR)₂

wherein R is a straight-chain or branched alkyl residue having 1 to 30 carbon atoms, a straight-chain or branched alkenyl residue having 2 to 30 carbon atoms, a monocyclic or polycyclic alkyl residue having 3 to 30 carbon atoms, a monocyclic or polycyclic alkenyl residue having 4 to 30 carbon atoms, or a monocyclic or polycyclic aromatic residue having 6 to 30 carbon atoms, these residues being optionally substituted by one or several substituents.

## Description

This invention relates to pharmaceutical preparations containing palladium complexes and the use thereof for treating tumoral and autoimmune diseases.

At present, cancerous diseases are usually treated by drug therapy or radiotherapy before and/or after surgery. Oncotherapy by drugs, i.e. chemotherapy, uses compounds influencing the cancer growth in various ways. However, chemotherapy is often accompanied by serious side-effects unpleasant for patients, such as hair loss, nausea, vomiting, tiredness, damage of bone marrow and white blood cells. This applies particularly to the platinum compounds used so far, such as cisplatin or carbo-platinum. More or less serious secondary infections also occur frequently. In addition, not all of the tumor kinds respond to chemotherapy, e.g. renal cell carcinoma or tumors of the gastro-intestinal tract.

Recently, there had been some promising results with platinum-xanthogenate complexes, called "thioplatin" (DE 199 40 407.0). However, some difficulties occurred when administering the maximum tolerable and maximum therapeutic dosage to the patient in view of the poor solubility of the compounds.

In the past autoimmune diseases have been usually treated with cytokines and heavy metal compounds. However, this treatment often has not been very successful and the autoimmune disease could not been stopped and the symptoms did not improve.

Thus, it is the object of the present invention to provide an effective drug for treating cancerous or autoimmune diseases. The drug shall be effective in little dosage, have as little toxic effect on healthy cells as possible and little side-effects. Moreover, the drug shall also be suitable for local therapy and be administrable by way of out-patient treatment. Besides, the drug shall also lower the risk of a relapse. In addition, it shall be possible to store the drug without loss of action over a prolonged period of time.

It was found surprisingly that palladium dithiocarboxylic acid complexes form stable compounds having an excellent anti-tumoral and anti-autoimmune effect.

Therefore, the subject matter of the invention relates to pharmaceutical preparations containing palladium complexes of the general formula (I)

Pd(S₂COR)₂ (I)

wherein R is a straight-chain or branched alkyl residue having 1 to 30 carbon atoms, a straight-chain or branched alkenyl residue having 2 to 30 carbon atoms, a monocyclic or polycyclic alkyl residue having 3 to 30 carbon atoms, a monocyclic or polycyclic alkenyl residue having 4 to 30 carbon atoms, or a monocyclic or polycyclic aromatic residue having 6 to 30 carbon atoms, these residues being optionally substituted by one or several substituents.

Any straight-chain or branched C1-30 alkyl residue may be used. Examples thereof are methyl, ethyl, n-propyl, isopropyl, n-butyl, isobutyl, sec.-butyl, tert.-butyl, n-pentyl, isopentyl, neopentyl, n-hexyl, 2-methylpentyl, 3-methylpentyl, 2,2-dimethylbutyl, 2,3-dimethylbutyl, n-heptyl, 2-methylhexyl, 3-methylhexyl, 2,2-dimethylpentyl, 2,3-dimethylpentyl, 3,3-dimethylpentyl, 3-ethylpentyl, 2,2,3-trimethylbutyl, n-octyl, 2-methylhepyl, 3-methylheptyl, 4-methylheptyl, 2,2-dimethylhexyl, 2,3-dimethylhexyl, 2,4-dimethylhexyl, 3,3-dimethylhexyl, 3,4-dimethylhexyl, 4,4-dimethylhexyl, 2-ethylhexyl, 3-ethylhexyl, 4-ethylhexyl, 2,2,3-trimethylpentyl, 2,3,3-trimethylpentyl, 3-methyl-3-ethylpentyl, etc. groups.

Because of their better solubility short alkyl chains, such as methyl, ethyl, propyl and isopropyl groups, are preferred.

R is preferably straight-chain C1-14 alkyl residues or C3-14 cycloalkyl residues. R preferably denotes -CH₃CH₂ or isopropyl.

Any straight-chain or branched C2-30 alkenyl residue can be used. Examples thereof are vinyl, propenyl, isopropenyl, allyl, 2-methylallyl, butenyl or isobutenyl, hexenyl or isohexenyl, heptenyl or isoheptenyl, octenyl, or isooctenyl groups. Vinyl, propenyl and isopropenyl groups are preferred.

The cycloalkyl residue having 3 to 30 carbon atoms may be any cycloalkyl residue. Examples thereof are cyclopropyl, cyclobutyl, cyclopentyl or cyclohexyl, cycloheptyl, cyclooctyl, cyclononyl or cyclodecyl groups. Cyclopropyl, cyclobutyl, cyclopentyl and cyclohexyl groups are preferred.

The cycloalkenyl residue having 4 to 30 carbon atoms may be any cycloalkenyl residue. Examples thereof are cyclobutenyl, cyclopentenyl or cyclohexenyl, cycloheptenyl, cyclooctenyl, cyclononenyl or cyclodecenyl groups. Cyclobutenyl, cyclopentenyl or cyclohexenyl groups are preferred.

Examples of polycyclic alkyl residues and alkenyl residues, respectively, are norbornane, adamantane or benzvalene.

R may also be any monocyclic or polycyclic C6-30 aryl residues. Examples thereof are a carbocyclic, monocyclic residue, e.g. the phenyl group; a heterocyclic, monocyclic residue, e.g. the groups thienyl, furyl, pyranyl, pyrrolyl, imidazolyl, pyrazolyl, pyridyl, pyrimidinyl, pyrazinyl, thiazolyl, oxazolyl, pyrrolinyl, imidazolinyl, pyrazolinyl, thiazolinyl, triazolyl, tetrazolyl, and the positional isomers of the heteroatom or heteroatoms which may comprise these groups, a residue consisting of carbocyclic anellated rings, e.g. the naphthyl group or the phenanthrenyl group, a residue consisting of anellated heterocyclic rings, e.g. benzofuranyl, benzothienyl, benzimidazolyl, benzothiazolyl, naphtho[2,3-b]thienyl, thianthrenyl, isobenzofuranyl, chromenyl, xanthenyl, phenoxathiinyl, indolizinyl, isoindolyl, 3H-indolyl, indolyl, indazolyl, purinyl, quinolizinyl, isoquinolyl, quinolyl, phthalzinyl, naphthyridinyl, quinoxalinyl, quinazolinyl, cinolinyl, pteridinyl, carbazolyl, &-carbolinyl, acridinyl, phenazinyl, phenothiazinyl, phenoxazinyl, indolinyl, isoindolinyl, imidazopyridyl, imidazopyridmidinyl or also the anellated polycyclic systems consisting of heterocyclic monocycles as defined above, such as furo[2,3-b]pyrrole or thieno[2,3-b]furane, and particularly the phenyl, furyl groups, such as 2-furyl, imidazolyl, such as 2-imidazolyl, pyridyl, such as 2-pyridyl, 3-pyridyl, 4-pyridyl, pyrimidinyl, such as pyridmidin-2-yl, thiazolyl, such as thiazol-2-yl, thiazolinyl, such as thiazolin-2-yl, triazolyl, such as triazolyl-2-yl, tetrazolyl, such as tetrazole-2-yl, benzimidazolyl, such as benzimidazole-2-yl, benzothiazolyl, benzothiazole-2-yl, purinyl, such as purin-7-yl or quinolyl, such as 4-quinolyl.

Preferably present substituents of the various above-mentioned residues may be selected from the following group:
- halogen: fluorine, chlorine, bromine, iodine,
- amino, alkylamino, dimethylamino or ethylamino, dialkylamino, such as dimethylamino, diethylamino, methylethylamino, each of these dialkylamino residues being optionally present in oxide form,
- amino alkyl such as aminomethyl or aminoethyl,
- dialkylaminoalkyl, such as dimethylaminomethyl or dimethylaminoethyl,
- dialkylaminoalkyloxy, such as dimethylaminoethyloxy,
- hydroxyl,
- free esterified carboxyl group, such as alkoxy carbonyl, e.g. methoxycarbonyl or ethoxycarbonyl, or converted into a salt, e.g. by a sodium or potassium atom,
- alkyl having 1 to 8 carbon atoms, such as methyl, ethyl, propyl, isopropyl, butyl, isobutyl, tert.-butyl, optionally substituted by one or several halogen atom(s), e.g. by fluorine, such as trifluoromethyl,
- oxo, cyano, nitro, formyl,
- acyl, such as acetyl, propionyl, butyryl, benzoyl,
- acyloxy, such as acetoxy or a residue of formula:

   -O-CO-(CH₂)ₙCO₂H,

   wherein n = 1 to 5,
- alkoxy, such as methoxy, ethoxy, propyloxy, isopropyloxy, butyloxy,
- alkylthio, such as methylthio, ethylthio, propylthio, isopropylthio, butylthio,
- carbamoyl,
- alkenyl, such as vinyl, propenyl,
- alkinyl, such as ethinyl, propinyl, and
- aryl, such as phenyl, furyl, thienyl.

An alkyl residue, substituted by one or several halogen atom(s), such as trifluoromethyl, trifluorobutyl, pentafluoropropyl, pentafluorobutyl, pentafluoropentyl, heptafluorobutyl, or nonafluorobutyl group or 2-chloroethyl can be mentioned as examples of such substituted residues.

All in all, compounds of above formula (I) can be described by the expression "thiopalladium compounds".

The compounds of formula (I) are preferably produced by a reaction of an alkaline O-alkyl dithiocarbonate with an dialkaline tetrachloropalladate (II) as described in C. G. Sceney, R. J. Magee, Palladium Xanthate Complexes Chimia Scripta, 1974, 6, 47-48; C. G. Sceney, J. O. Hill, R. J. Magee, Thermal Studies on palladium alkyl xanthates, Thermochimica Acta 1973, 6, 111-117 and G. W. Watt, B. J. McCormick, The synthesis and characterization of methyl and ethylxanthato complexes of Pt(II), Pd(II), Ni(II), Cr(III) and Co(III), J. Inorg. Nucl. Chem. 1965, 27, 898-900.

The compounds of formula (I) are suitable for treating various cancerous diseases, such as testicular tumors, ovarian carcinomas, bladder carcinomas, colonic carcinomas, prostatic carcinomas, parvocellular and non-parvocellular bronchial carcinomas, carcinomas of the cephalic and cervical parts, carcinomas of the thoracic and abdominal regions, cervical and endometrial carcinomas, sarcomas and melanomas as well as leukemias. The treatment of the parvocellular bronchial carcinoma or colorectal carcinoma is preferred. The treatment can also be carried out as a treatment associated with a radiotherapy or before and/or after a surgery.

The compounds of formula (I) are well tolerable. The L50 value is lower by a factor of 3 than that for the cis-platin known in tumor treatment. When a dosage having good antitumoral effects is used, hardly any side-effects occur. In particular, the feared nephrotoxicity known for cisplatin has not yet occurred in this way in the case of the present compounds. Another advantage of the compounds according to the invention is that they have a broad activity spectrum against the most varying tumors and are particularly also effective against tumors which have resisted treatment with platinum compounds (e.g. cisplatin) so far. The palladium compounds are particularly suitable for solid tumors. It has been found out that their cytotoxic effect to human tumor cell lines is 30-50 fold higher than that of cisplatin. In comparison to thioplatin compounds the cytotoxic effect of the palladium compounds is 7 fold higher. In view of the low toxicity it is possible to achieve the maximum tolerable dose (100 mg/kg when administered orally) without any problems.

Heavy metal compounds such as gold complexes are often used for the treatment of autoimmune diseases. These compounds have severe side effects, such as nephrotoxicity and bone marrow depression. The palladium complexes of the present invention, however, are well tolerated and therefore are superior to the currently available heavy metal drugs. The compounds of formula (I) can be used e.g. for the treatment of arthritis, Morbus Crohn, colitis ulcerosa, diabetes, multiple sclerose, Lupus erytematosis.

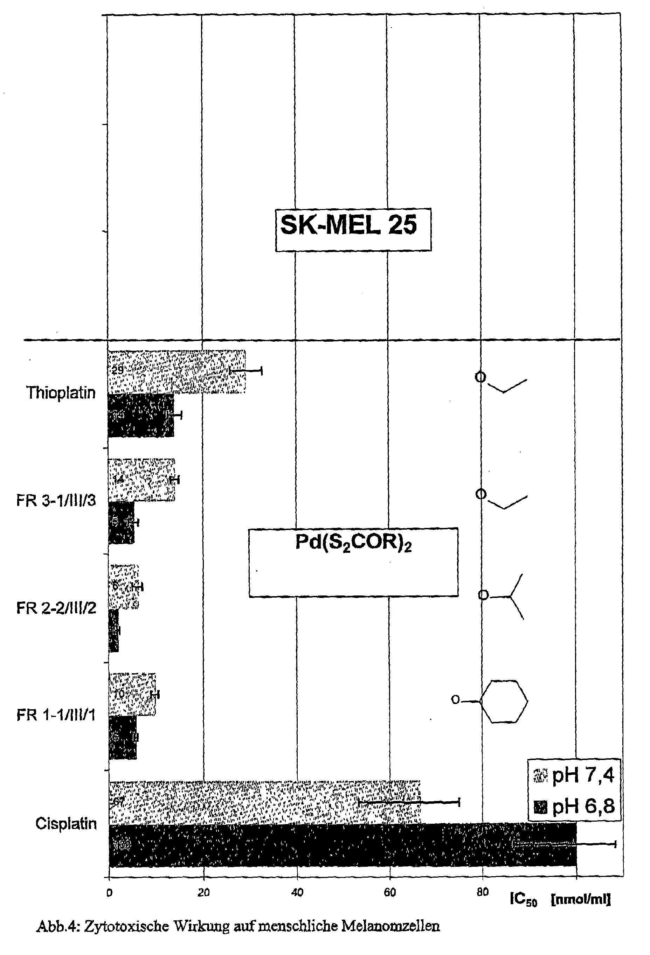

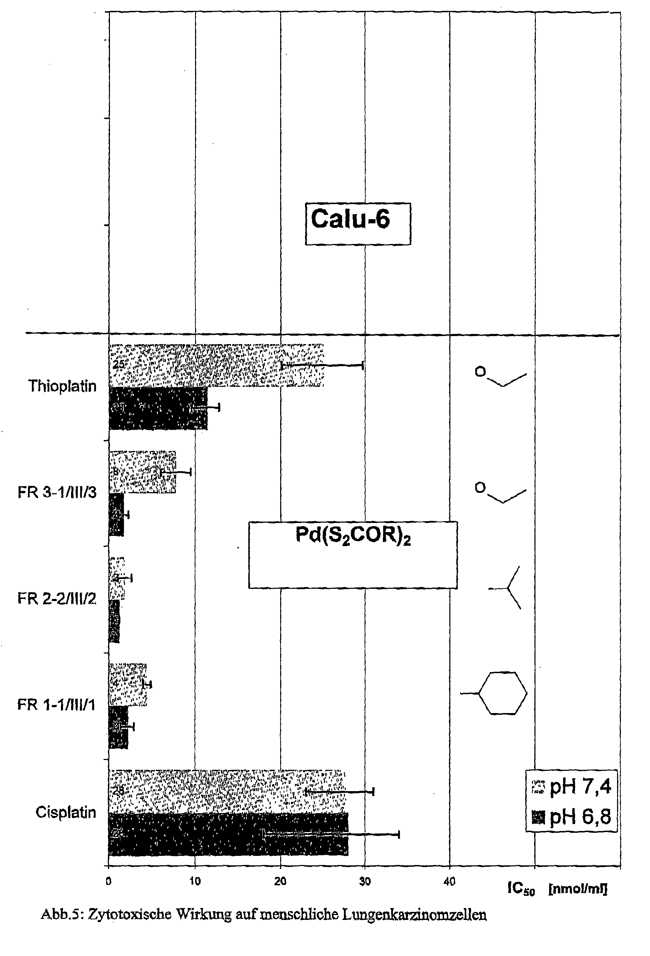

Another advantage consists in that the effectiveness of the compounds according to the invention is greater in the slightly acidic pH range than in the alkaline one, since many tumor tissues have a rather acidic environment. The inventors carried out investigations (c.f. Figs. 4 and 5) with bis[O-isopropyldithio-carbonato]palladium (II), bis[O-cyclohexal-dithiocarbonato]palladium (II) and bis[O-ethyldithiocarbonato]palladium (II), all palladium coordination complexes according to formula (I) in which palladium is complexed with sulfur atoms. Following protonation, two sulfur ligate ions open reversibly (so that an aqua complex forms) which can initiate a cross-linkage of DNA. After raising the pH value, the protons dissociate from the sulfur atoms and the inert molecule is recovered. The shift of the pH value to the (slightly) acidic range from the prodrug according to formula (I) effects the formation of the actually reactive compound. This concept is confirmed by the examples.

The pharmaceutical preparation according to the invention can be administered in various ways, e.g. orally, parenterally, cutaneously, subcutaneously, intravenously, intramuscularly, rectally or intratuomorally. The intravenous or intratumoral administration is preferred, i.e. the administration in certain diseased organs or parts of the body. The pharmaceutical preparation is administered to a patient over a period to be determined by a physician. The pharmaceutical preparation can be administered to both human beings and mammals.

The dosage of the compound according to the invention is determined by a physician by means of the patient-specific parameters, such as age, weight, sex, severity of the disease, etc. The dosage is preferably from 0.001 to 1000 mg/kg body weight.

In accordance with the kind of administration, the pharmaceutical preparation is formulated in a suitable way, e.g. in the form of simple or coated tablets, hard or soft gelatin capsules, powders for reconstitution prior to use, granular powders, suppositories, ovules, injectables, infusion solutions, pomades, creams, gels, microspheres, implants, which are produced according to conventional galenic processes.

The compounds of formula (I) can optionally be administered together with further active substances and with excipients common in pharmaceutical compositions, e.g. depending on the preparation to be produced talcum, gum arabic, lactose, starch, magnesium stearate, cocoa butter, aqueous and non-aqueous carriers, adipoids of animal or vegetable origin, paraffin derivatives, glycols (particularly polyethylene glycol), various plasticizers, dispersants or emulsifiers, preservatives.

Additives such as sodium chloride solution, ethanol, sorbitol, glycerol, olive oil, almond oil, propylene glycol or ethylene glycol can be used for the production of liquid preparations.

Infusions or injectable solutions are preferably produced. They are preferably aqueous solutions or suspensions, it being possible to produce them prior to use, e.g. from lyophilized preparations containing the active substance as such or together with a carrier such as mannitol, lactose, glucose and the like. The ready-to-use solutions are sterilized and optionally mixed with adjuvants, e.g. preservatives, stabilizers, emulsifiers, solution aids, buffers and/or salts for controlling the osmotic pressure. Sterilization can be achieved by sterile filtration through filters having a small pore size, whereupon the composition can optionally be lyophilized. Small amounts of antibiotics can also be added so as to maintain sterility.

The provision of the pharmaceutical preparation according to the invention in a unit dosage form for administration to a mammal requiring anticancer treatment or treatment against an autoimmune disease is advantageous.

The invention also relates to pharmaceutical preparations and pharmaceutical compositions, respectively, which contain a therapeutically effective amount of the active ingredient (compound of formula (I) according to the invention) together with organic or inorganic inert solid or liquid pharmaceutically compatible carriers and diluents, respectively, which are suited for the intended administration and which show no unfavorable interactions with the active ingredients.

The invention also relates to a process for the production of a pharmaceutical composition, which is characterized by mixing the compound according to formula (I) with a pharmaceutically compatible carrier.

The drugs according to the invention may include particularly the compounds described in the experimental part and more particularly the compounds in which in above formula (I) R is a methyl, ethyl, propyl or isopropyl group, or a CH₂CH₂OH, CH₂CH₂CH₂OH or CH₂(CH₂)₂CH₂OH group.

The pharmaceutical preparations and/or pharmaceutical compositions according to the invention comprise as active substance at least one active substance as defined above. Optionally further pharmaceutical active substances can be added to the composition, such as immunosuppressive agents, e.g. cyclosporine, rapamycin, 15-deoxyspergualine, OKT3, azathioprine; cytokines (e.g. TNF), interferon, etc. In addition, the composition according to the invention can additionally contain a steroid or further cytostatic agents (e.g. cisplatin, methotrexate, aminopterin, dacarbacine, nitroso urea compounds, fluorouracil, bleomycin, daunomycin, daunorubicin, doxorubicin, mithramycin, mitomycin C, etc.).

The invention is further explained by the figure:
- Fig. 1: Antitumoral activity of Bis(O-isopropyl-dithiocarbonato)palladium (II) in vivo
- Fig. 2: Toxicity after i.v. application of 20 mg/kg of Bis(O-isopropyl-dithiocarbonato)palladium (II)
- Fig. 3: Toxicity after oral application of 100 mg/kg and 200 mg/kg Bis(O-isopropyl-dithiocarbonato)palladium (II)
- Fig. 4: Cytotoxic effect on human melanoma cell line SK-MEL25
- Fig. 5: Cytotoxic effect on human lung carcinoma cell line Calu-6

The invention is explained in more detail by the below examples.

### Example 1: Synthesis of palladium complexes

### General procedure:

All O-alkyl-dithiocarbonates were prepared according to methods as described in C. G. Sceney, R. J. Magee, Palladium Xanthate Complexes Chimia Scripta, 1974, 6, 47-48; C. G. Sceney, J. O. Hill, R. J. Magee, Thermal Studies on palladium alkyl xanthates, Thermochimica Acta 1973, 6, 111-117 and G. W. Watt, B. J. McCormick, The synthesis and characterization of methyl and ethylxanthato complexes of Pt(II), Pd(II), Ni(II), Cr(III) and Co(III), J. Inorg. Nucl. Chem. 1965, 27, 898-900.

Potassium O-alkyl-dithiocarbonates (KS₂COR) were reacted with dipotassium tetrachloropalladate(II) (K₂PdCl₄) by the following general procedure:
1.53 mmol K₂PdCl₄ in 5 ml dest. H₂O was mixed with 4.60 mmol of the potassium O-alkyl-dithiocarbonates (KS₂COR) in 5 ml H₂O dest. under vigorous stirring, followed by an additional 5 ml of dest. H₂O. The reaction vessel was closed and the mixture was stirred for 80-120 min at room temperature. The precipitate was separated by suction filtration, washed three times with dest. H₂O followed by a wash with either diethylether or pentane. Re-crystallisation from acetone or acetone/CHCl₃ yielded the Pd-complexes as yellow or brown crystals. The product was dried in a vacuum (10-3 Torr) for 1-2 days.

The structures of the bis(O-alkyl-dithiocarbonato)-palladium(II)-complexes (1-7) were verified by ¹H- and ¹³C-NMR-, IR- and UV-spectroscopy, by mass spectrometry and elemental analysis.

The Pd-complexes are insoluble in water, poorly soluble in acetone and soluble in CHCl₃.
Bis(O-cyclohexyl-dithiocarbonato)palladium(II) (1)
   yield: 82%, brown crystalls .
Bis(O-isopropyl-dithiocarbonato)palladium(II) (2)
   yield: 79%, orange crystalls.
Bis(O-ethyl-dithiocarbonato)palladium(II) (3)
   yield: 79%, brown-gold crystalls.
Bis(O-(2-methyl)-butyl-dithiocarbonato)palladium(II) (4)
   yield: 18%, orange-brown crystall).
Bis(O-butyl-dithiocarbonato)palladium(II) (5)
   yield: 57%, brown crystalls.
Bis(O-hexyl-dithiocarbonato)palladium(II) (6)
   yield: 64%, orange-gold crystalls.
Bis(O-methyl-dithiocarbonato)palladium(II) (7)
   yield; 85%, red crystalls.

### Example 2: Anti-tumoral activity of Pd-complexes (1-7) on human cancer cell lines

### Cytotoxicity assay

The Pd-complexes were dissolved in acetone (1 mg/ml stock solution). Human cancer cell lines SK-MEL 25 (melanoma) and CALU-6 (lung cancer) were used for the determination of the cytotoxic activity of Pd-complexes. For comparison, the well known anti-tumor drugs cisplatin and corresponding platinum complexes were included. Cells were seeded at a density of 2x10⁶ cells/ 96 well plate. After 24 h, tissue culture medium was removed and fresh medium supplemented with 10% fetal calf serum and 100 units/ml penicillin/streptomycin containing either 2.2 g/l (pH 7.4) or 0.85 g/l (pH 6.8) NaHCO₃ was added. After 1 hour of equilibration in an atmosphere containing 5% CO₂, drugs were added at various concentrations (50, 25, 12.5, 6.25, 3.1, 1.6, 0.8 µg/ml) in quadruplicate. Untreated cultures served as controls. Two hours later, the culture medium was replaced with 0.1 ml of fresh tissue culture medium (pH 7.4, 10% fetal calf serum). Culture plates were then incubated for 48 h (SK-MEL 25) or 72 h (Calu-6) at 37°C. Then, cells were fixed with 3% formaldehyde and stained with 1% crystalviolet. The number of viable cells is proportional to the amount of bound cristalviolet. Therefore, the relative cell number could be determined at an Antos 2001 ELISA reader (550 nm). Dose response curves were established from the OD data and the concentration at which the cell number was reduced to 50 % of the control (IC50) was determined. Results are shown in table 1 and Figs. 4 and 5.

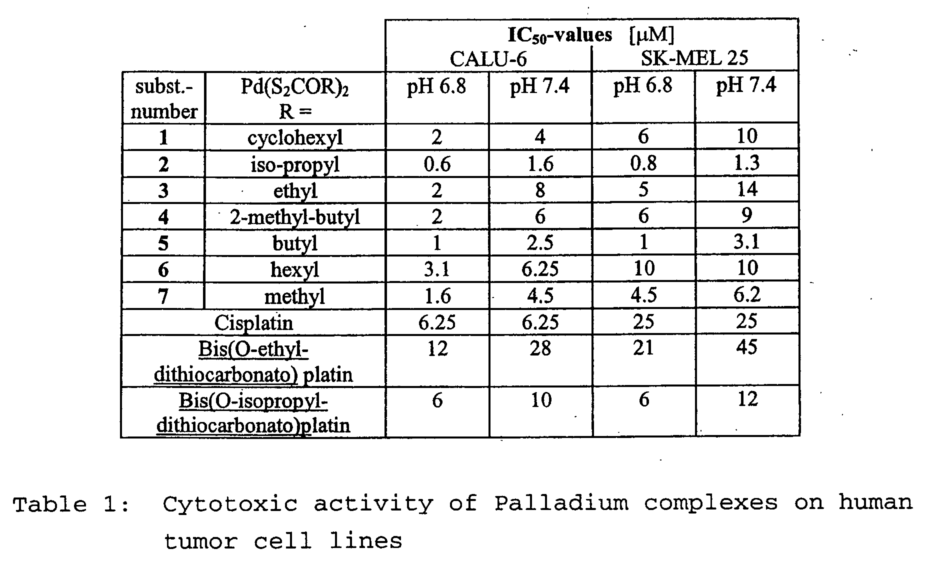

### Example 3: Anti-tumoral activity of Bis(O-isopropyl-dithiocarbonato)palladium(II) in vivo

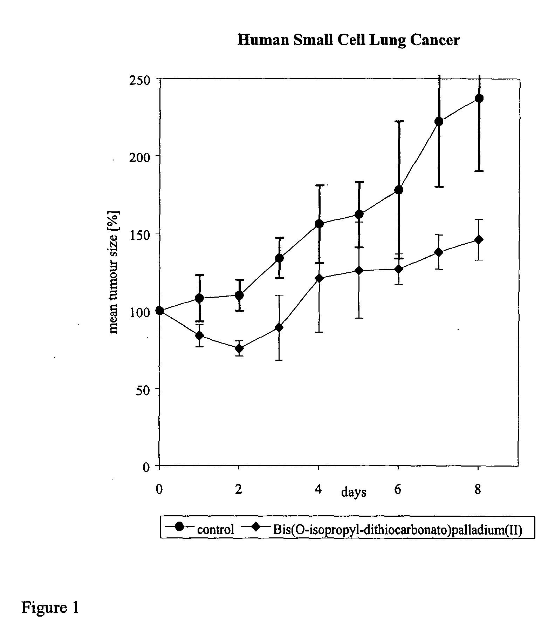

Human small cell lung cancer cells (H10) were transplanted in athymic nude mice (strain Balb c). Three weeks later, when tumors had grown to a size of 6-10 mm treatment was initiated. Five animals recieved a single i.v injection of Bis(O-isopropyl-dithiocarbonato)palladium(II) (0.2 mg/ml in 30 % Cyclodextrin, 0.2 ml/20 g body weight) at day 0 and at day 4. Tumor size was determined in two dimensions, using callipers. Ten animals received 30 % cyclodextrin as a control. Relative tumor growth was determined daily. Mean values ± standard deviation are indicated in figure 1.

### Example 4: Toxicity studies

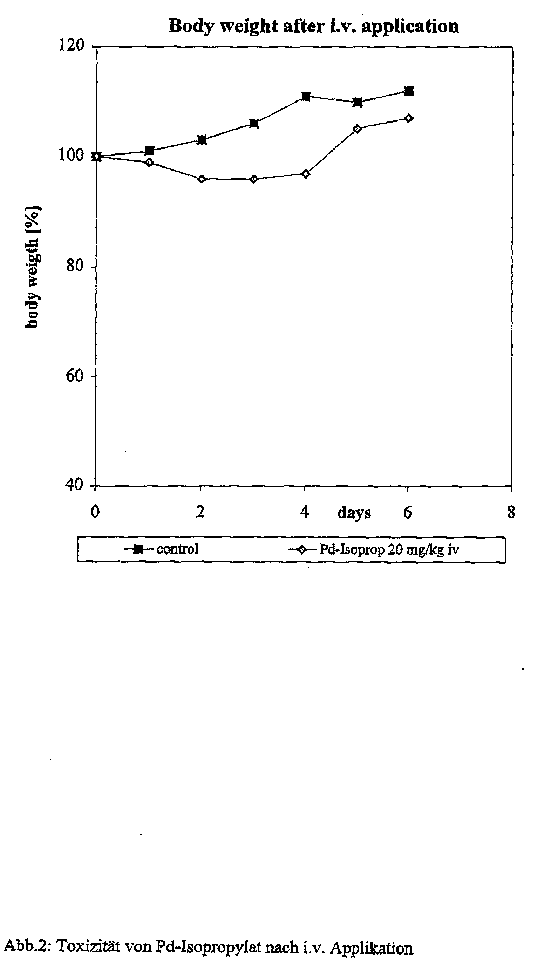

Female Balb c mice (6-8 weeks old, 5 animals/group) were injected (i.v. in lateral tail vein) with 0.2 ml/20g body weight of a solution of 2 mg/ml Bis(0-isopropyl-dithiocarbonato) palladium (II) in 30% cyclodextrin/70% water. Control animals received 30% cyclodextrin/70% water. Mean body weight of each group was determined daily. Standard deviations were found to be smaller than 5%. The results are shown in Fig. 2.

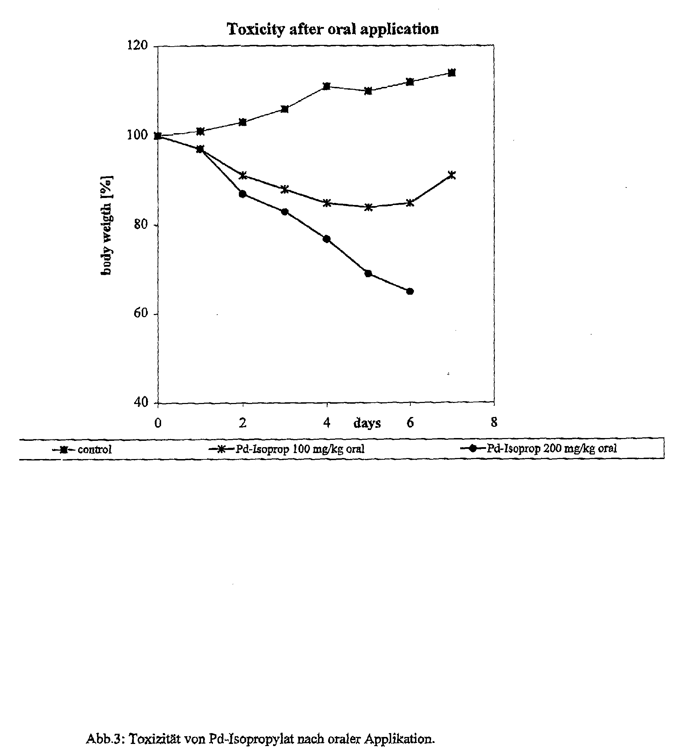

Femals Balb c mice (6-8 weeks old, 5 animals/group) received (by intragastral intubation) 0.2 ml peanut oil/20g body weight containing either 0, 10 or 20 mg/ml Bis(O-isopropyl-dithiocarbonato) palladium (II). Mean body weight of each group was determined daily. Standard deviations were found to be smaller than 8%. The results are shown in Fig. 3.

## Claims

1. A pharmaceutical preparation **characterized by** a content of at least one compound of general formula (I)
Pd(S₂COR)₂
wherein R is a straight-chain or branched alkyl residue having 1 to 30 carbon atoms, a straight-chain or branched alkenyl residue having 2 to 30 carbon atoms, a monocyclic or polycyclic alkyl residue having 3 to 30 carbon atoms, a monocyclic or polycyclic alkenyl residue having 4 to 30 carbon atoms, or a monocyclic or polycyclic aromatic residue having 6 to 30 carbon atoms, these residues being optionally substituted by one or several substituents.

2. The pharmaceutical preparation according to claim 1, wherein in the compound of formula (I) R is a straight-chain C1-14 alkyl residue or a C3-14 cycloalkyl residue each.

3. The pharmaceutical preparation according to claim 1 or 2, in the compound of formula (I) wherein R is CH₃CH₂, isopropyl, CH₂CH₂OH, CH₂CH₂CH₂OH or CH₂(CH₂)₂CH₂OH.

4. The pharmaceutical preparation according to any one of claims 1 to 3, wherein the compound of formula (I) is Bis(O-cyclohexyl-dithiocarbonato)palladium(II), Bis-isopropyl-dithiocarbonato)palladium(II), Bis(O-ethyl-dithiocarbonato)palladium(II), Bis(O-(2-methyl)-butyl-dithiocarbonato)palladium(II), Bis(O-butyl-dithiocarbonato)-palladium(II),Bis(O-hexyl-dithiocarbonato)palladium(II) or Bis(O-methyl-dithiocarbonato)palladium(II).

5. The pharmaceutical preparation according to any one of claims 1 to 4, comprising additionally an immunosuppressive compound selected from the group consisting of cyclosporine, rapamycin, 15-deoxyspergualine, OKT3 and azathioprine.

6. The pharmaceutical preparation according to any one of claims 1 to 4, comprising additionally cytokines, interferon or further cytostatic agents.

7. The pharmaceutical preparation according to any one of claims 1 to 6, provided in a unit dosage form for administration to a mammal which requires treatment with an anticancer or anti-autoimmunic agent.

8. The pharmaceutical preparation according to any one of claims 1 to 7, further comprising a pharmaceutically compatible inert carrier or a diluent.

9. Use of a pharmaceutical preparation according to any one of claims 1 to 8 for treating a cancerous disease.

10. Use according to claim 9, wherein the cancerous disease is the parvocellular bronchial carcinoma or colorectal carcinoma.

11. Use of a pharmaceutical preparation according to any one of claims 1 to 8 for treating an autoimmune disease.

12. A process for the production of a pharmaceutical preparation according to any one of claims 1 to 8, **characterized in that** the compound according to formula (I) is mixed with a pharmaceutically compatible carrier or diluent.
